**Europäisches Patentamt**

**European Patent Office**

**Office européen des brevets**

(19)

(11) Publication number : **0 310 646 B1**

# EUROPEAN PATENT SPECIFICATION

(12)

(45) Date of publication of patent specification :
26.08.92 Bulletin 92/35

(51) Int. Cl.⁵ : **A61M 5/00, A61M 5/32**

(21) Application number : 88903321.3

(22) Date of filing : 22.04.88

(86) International application number :
PCT/GB88/00308

(87) International publication number :
WO 88/08313 03.11.88 Gazette 88/24

(54) **NEEDLE DISPOSAL SYSTEM.**

(30) Priority : 22.04.87 GB 8709453

(43) Date of publication of application :
12.04.89 Bulletin 89/15

(45) Publication of the grant of the patent :
26.08.92 Bulletin 92/35

(84) Designated Contracting States :
AT BE CH DE FR IT LI LU NL SE

(56) References cited :
FR-A- 2 586 566
FR-A- 2 586 568
US-A- 4 565 311
US-A- 4 576 281

(73) Proprietor : **McCAMMON, John Wesley**
**104 Killinchy Road**
**Comber BT23 5NE (GB)**
Proprietor : **MOFFET, Trevor Lonsdale**
**160 Donaghadee Road**
**Newtownards, County Down BT23 3QP (GB)**

(72) Inventor : **McCAMMON, John Wesley**
**104 Killinchy Road**
**Comber BT23 5NE (GB)**
Inventor : **MOFFET, Trevor Lonsdale**
**160 Donaghadee Road**
**Newtownards, County Down BT23 3QP (GB)**

(74) Representative : **Connor, Terence Kevin**
**T.K. Connor & Co. 19 Station Road**
**Sidcup, Kent DA15 7EB (GB)**

## Description

This invention relates to a needle disposal system for use in disposing of used hypodermic needles from syringes in a manner to reduce or avoid the danger of any infection, such as hepititis or aids, being transmitted.

Needle disposal systems have been proposed heretofore. FR-A-2 586 566 discloses a system to allow needles to be unsheathed and resheathed safely after use. The system has an inlet in its top face and a first cap with an aperture alignable with said inlet, and through which aperture and inlet a sheath can be located into which a used needle can be fed, the sheathed needle thereafter being removed out through the inlet for disposal. The system is adapted to be hand held. The first cap has an inverted skirt extending outwardly and upwardly to act as a protective shroud to protect a hand when holding said assembly during a needle inserting operation. US-A-4576 281 and US-A-4 565 311 also disclose needle disposal systems.

According to the present invention, a needle disposal system comprises an assembly adapted to be hand held, the assembly having a first cap, a head unit and a disposable container for holding a plurality of used needles, the first cap having an aperture, the head unit having a passage formed by aperture means incorporating a valve device and the container having an inlet provided in a top end thereof, characterised in that the the head unit is formed in two parts with a first part having two spaced plates in which aligned apertures are provided and a second part being a trigger mechanism movable between the two plates and having a passageway alignable with the aligned apertures, the aperture and the aligned apertures and the inlet being in alignment when the components are assembled together, the passageway of the trigger mechanism being biased to an out-of-alignment position whereby on the passageway being moved into alignment with said apertures a needle can be inserted into the aperture to pass into the container, the container having means to prevent the return of a needle through the inlet.

Preferably, the first cap has an inverted skirt extending outwardly and upwardly beyond the sides of the head unit to act as a protective shroud to protect a hand when holding the head unit of said assembly during a needle inserting operation.

Preferably also, the trigger mechanism is spring-biased and has two plates in sliding relationship with spaced plates respectively and between which two plates the passageway extends, the two plates having apertures. The valve device is preferably formed by the aperture in the top plate of the two spaced plates, and the aperture in the upper of the two plates. Both apertures of the valve device are desirably provided with a series of serrations around opposed semi-circular parts thereof.

The container is preferably of a material resistant to passage therethrough of a needle. The head unit is beneficially reusable and of a material capable of being autoclaved.

A second cap in the form of a closure is desirably provided to be securable over said container after the head unit and/or first cap is removed.

An embodiment of the present invention will now be described, by way of example, with reference to the accompanying schematic drawings, in which:-

Fig. 1 is a side view of a needle disposal system according to the present invention;

Fig. 2 is a plan view of Fig. 1;

Figs. 3A and 3B are plan views respectively of a head unit of the system and of a top plate of a trigger mechanism;

Fig. 4 is a vertical cross-sectional view of the system shown in Fig.1 through its transverse axis;

Fig. 5 is a vertical cross-sectional view of the system through its longitudinal axis with the trigger mechanism shown in a closed position;

Fig. 6 is a vertical cross-sectional view of the system through its longitudinal axis with the trigger mechanism shown in a compressed open position;

Fig. 7 is a similar view of a needle disposal system as shown in Fig. 4 with a syringe and needle located therein and located in a reclined rest position;

Fig. 8 is a cross-sectional view of a second cap of the system.

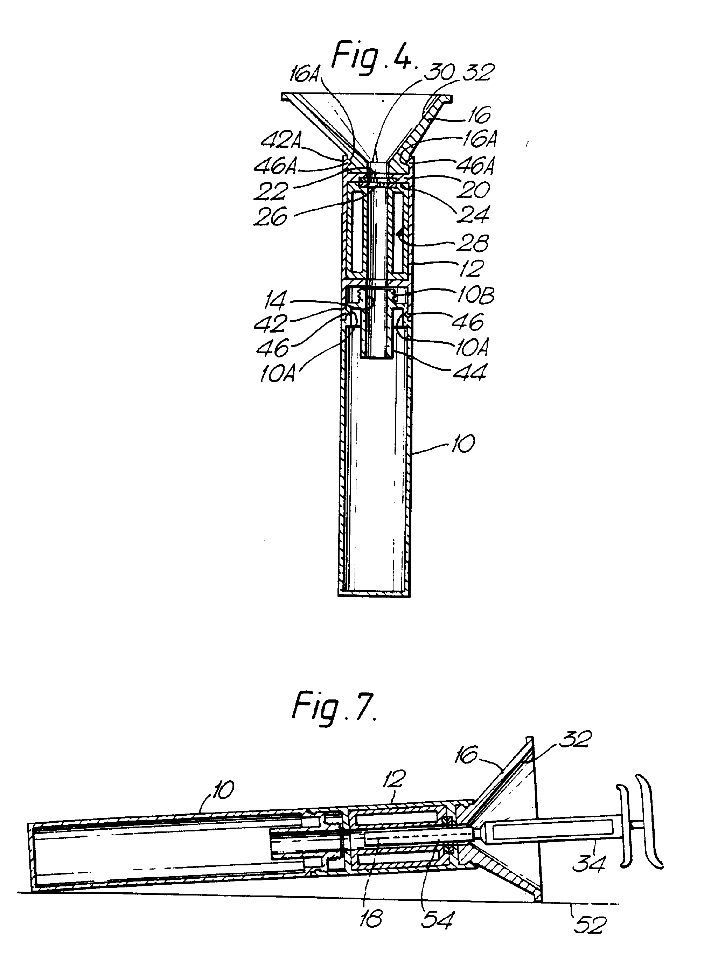

Referring to the drawings, a needle disposal system comprises an assembly adapted to be hand held in an upright manner and in which position the assembly will hereinafter be described. The assembly is of a disposable container 10 having an inlet in its top face and a head unit 12 securable over said top face. The container 10 and head unit 12 have a similar oblong horizontal cross-section. The container 10 has an external screw-thread 10B around a neck 14 of its inlet which stands proud of its top face as shown, and the head unit 12 has a depending surround 42 shaped as an extension of the container as shown in Figs. 4, 5, 6 and 7. Adjacent to the bottom edge of said surround 42 on each lateral side thereof, an elongate protuberance 46 is provided to engage in a complementary groove 10A around the lateral sides of the container 10 as shown in Fig. 4 to secure the container 10 and head unit 12 together. A similar surround 42A extends upwardly and on each lateral side an elongate protuberance 46A is provided for a purpose to be described hereinafter.

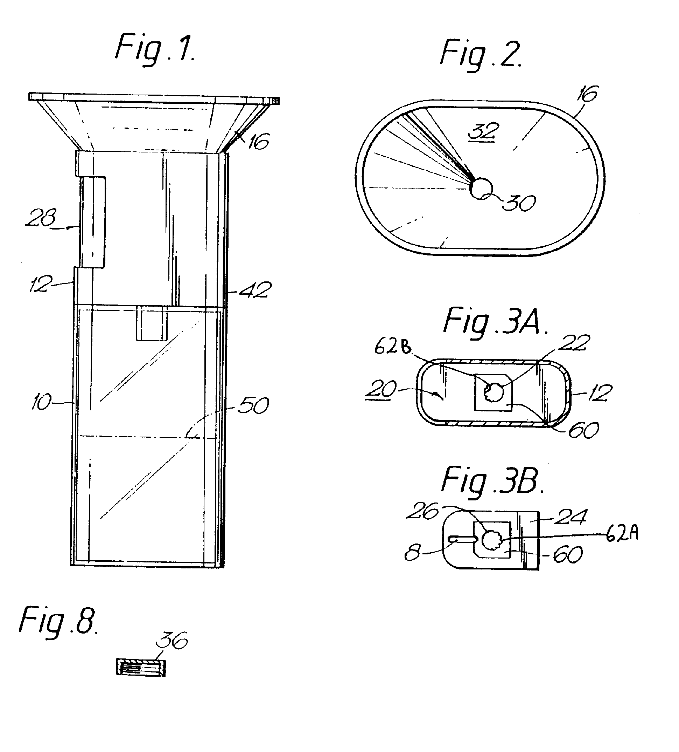

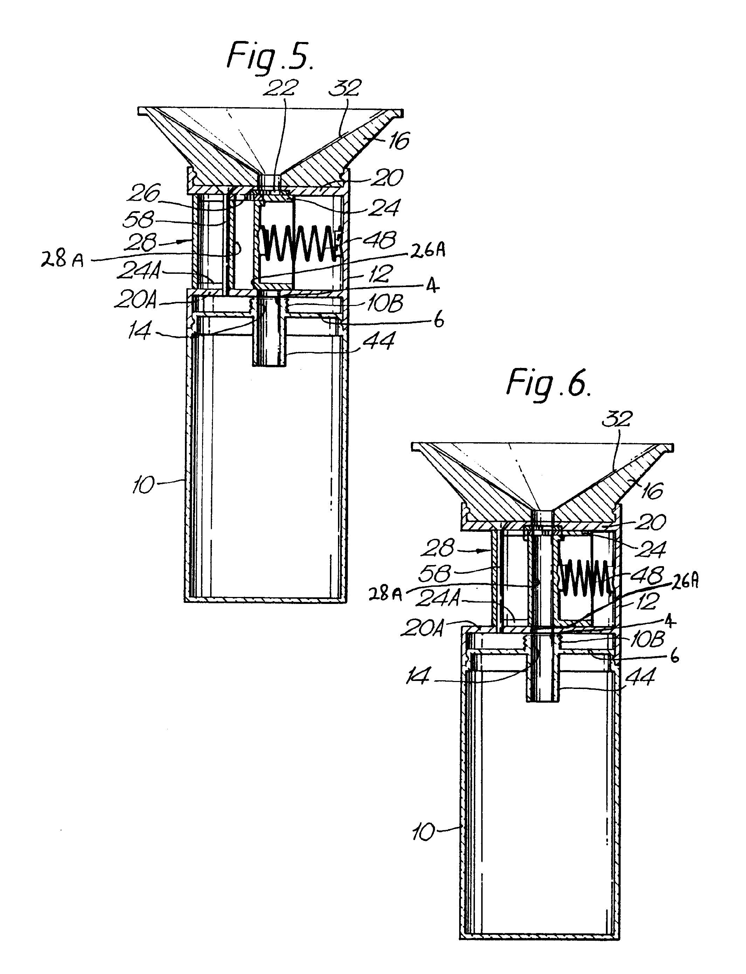

The head unit 12 has aperture means through which used needles can be fed into said container 10 for non-returnable passage therefrom. A tubular extension 44 projects from the top of the container 10 internally thereof as shown to provide the non-returnability of used needles fed into the container. The aperture means incorporates a valve device (Figs. 3A and 3B) formed by a circular aperture 22 being provided in top plate 20 and a plate 24 constrained to move horizontally parallelly to said plate 20 between two extreme position to respectively close off said aperture 22 in the top plate 20 and to open said aperture 22. The plate 24 has an aperture 26 alignable with aperture 22 of the top plate 20 when the plate 24 is in the open position. Aperture 26 is circular. Both apertures 22 and 26 are provided with a series of serrations as shown around opposed semi-circular parts thereof to form a complete circle of serrations. The plate 24 is spring-biased to the closed position. The plate 24 forms the top of a trigger of a trigger mechanism 28 which is constrained to move in the head unit 12 between inner and outer positions as shown in Figs. 5 and 6 respectively. The extent of movement of the trigger is determined by slot 8 (Fig. 3B) in plate 24, and a corresponding slot in a bottom plate 24A of the trigger, the slots beingengaged by a pin 58 (Figs. 5 and 6) secured in the head unit 12 between top plate 20 and a bottom plate 20A. The trigger mechanism 28 includes a helical spring 48 mounted between the inner wall of the head unit 12 and the inner wall of the trigger as shown. The spring 48 which forms the spring-biasing of plate 24 and movement of the plate 24 against its biasing is controlled by the trigger mechanism 28.

A first cap 16, shaped as shown in Fig. 2, is securable over said head unit 12 with an aperture 30 alignable with aperture 22 in said aperture means, the cap 16 having an inverted skirt 32 extending outwardly and upwardly to act as a protective shroud to protect a hand when holding said assembly during a needle inserting operation. The cap 16 has a groove 16A around the lateral sides of the bottom as shown in Fig. 4 to secure the cap 16 and the head unit 12 together.

The container 10 is commonly called a 'sharpes box' and is of a material, such as a synthetic plastics material, resistant to passage therethrough of a needle and is for disposal when full, while the head unit 12 is reusable and of a material capable of being autoclaved for sterilisation. In Fig. 1, there is shown in broken line the level 50 at which the container 12 is to be considered full. The container 12 is preferably of a transparent material so as to ascertain when the container 12 is full.

A second cap 36 shown in Fig. 10 and in the form of a closure is provided with an internal screw-thread to be securable over the neck 14 of said container 10 after the head unit 12 is removed.

The apertures 22, 26 are provided in metal plates 60 inset into complementarily shaped rebates formed in plates 20, 24.

In use, the assembly is held in one hand and a syringe 34 with needle 18 to be removed is held in the other hand. The trigger mechanism 28 is pressed to move plate 24 until its apertures 26 aligns with aperture 22 and the needle is then inserted the aligned apertures 22, 26 until the hub of the needle 18 is located below the plate 24. The trigger is released such that the nozzle of the syringe above the hub is clamped by the serrated edges of the two apertures 22, 26. By pulling or unscrewing the syringe 34 out of the assembly, the needle is released by the hub being prevented from outward movement by its contact under the plates 20, 24. The needle 18 then falls into the container 10. In like manner, other needles 18 can be disposed of into the container 10 until it is full or at the end of a particular time period of use of the syringe, at which time the container 10 can be detached from the head unit 12, a second cap 36 fitted thereto, and disposed of to waste. In the case where the needle being used is a multi-use needle ie. multi-use on the same patient, there is normally a sheath 54 provided. In this case, the sheath 54 can be clamped by the serrations on the apertures 22, 26, the assembly left on a surface 52 as shown in Fig. 7, and the needle 18 inserted into the sheath 54 between uses. After finishing using the syringe 34 on the patient, the needle 18 can be fully located in the sheath 54, the assembly lifted, the trigger pressed to release the sheath 54 and allow the sheath 54 and needle 18 to pass further into the container 10 and the sheath and needle detached in the same manner as hereinabove described and deposited into the container 10.

In a first modification, the aperture 26 may have a straight knife edge in place of the serrations and be used to guillotine off the needles from their roots in the respective hubs. This modification may be beneficial to people suffering from diabetes who have to inject themselves periodically and then have the problem of having to dispose of the used needles.

In a second modification, other means for securing the first cap 16, head unit 12 and container 10 can be used, such as moulded press stud fastenings.

## Claims

1. A needle disposal system comprising an assembly adapted to be hand held, the assembly having a first cap (16), a head unit (12) and a disposable container (10) for holding a plurality of used needles, the first cap (16) having an aperture (30), the head unit (12) having a passage formed by aperture means incorporating a valve device and the container (10) having an inlet (4) provided in a top end (6) thereof, characterised in that the head unit (12) is formed in two parts with a first part having two spaced plates (20, 20A) in which aligned apertures (22, 22A) are provided and a second part being a trigger mechanism (28) movable between the two spaced plates (20, 20A) and having a passageway (28A) alignable with the aligned apertures (22, 22A), the aperture (30) and the aligned apertures (22, 22A) and the inlet (4) being in alignment when the components are assembled together, the passageway (28A) of the trigger mechanism (28) being biased to an out-of-alignment position whereby on the passageway (28A) being moved into alignment with said apertures (22, 22A) a needle can be inserted into the aperture (30) to pass into the container (10), the container (10) having means (44) to prevent the return of a needle through the inlet (4).

2.  A needle disposal system as claimed in Claim 1, characterised in that the first cap (16) has an inverted skirt (32) extending outwardly and upwardly beyond the sides of the head unit (12) to act as a protective shroud to protect a hand when holding the head unit (12) of said assembly during a needle inserting operation.

3.  A needle disposal system as claimed in Claim 2, characterised in that the skirt (32) is oblong in plan and has one longitudinal side with a steeper incline than the opposite longitudinal side.

4.  A needle disposal system as claimed in any one of the preceding Claims, characterised in that the trigger mechanism (28) is spring-biased.

5.  A needle disposal system as claimed in Claim 1 or 4, characterised in that the trigger mechanism has two plates (24, 24A) in sliding relationship with plates (20, 20A) respectively and between which two plates (24, 24A) the passageway (28A) extends, the two plates (24, 24A) having apertures (26, 26A).

6.  A needle disposal system as claimed in Claim 5, characterised in that the valve device is formed by the aperture (22) in the top plate (20) of the spaced plates (20, 20A), and the aperture (26) in the upper of the two plates (24, 24A).

7.  A needle disposal system as claimed in Claim 6, characterised in that both apertures (22, 26) of the valve device are provided with a series of serrations (62, 62A) around opposed semi-circular parts thereof.

8.  A needle disposal system as claimed in Claim 7, characterised in that rebates are formed in the top and upper plates (20, 24) to receive metal plates (60) in which the apertures (22, 26) with the serrations (62. 62A) are provided.

9.  A needle disposal system as claimed in any one of the preceding Claims, characterised in that the extent of movement of the trigger mechanism (28) is determined by slots (8) through which a stationary pin (58) extends and is anchored in the spaced plates (20, 20A).

10. A needle disposal system as claimed in any one of Claims 2 to 9, characterised in that the first cap (16), the head unit (12) and the container (10) have cooperating male and female connection parts between adjoining components.

11. A needle disposal system as claimed in any one of the preceding Claims, characterised in that the prevention means is a tubular extension (44) projecting inwardly from around the inlet (4) of the container (10).

12. A needle disposal system as claimed in any one of the preceding Claims, characterised in that the container (10) is of a material resistant to passage therethrough of a needle.

13. A needle disposal system as claimed in any one of the preceding Claims, characterised in that the head unit (12) and the first cap (16) are reusable and of a material capable of being autoclaved.

14. A needle disposal system as claimed in any one of the preceding Claims, characterised in that the container (10) and head unit (12) are of plastics material.

15. A needle disposal system as claimed in any one of the preceding Claims, characterised in that a second cap (36) in the form of a closure is provided to be securable over the top of said container (10) after use and prior to disposal.

**Patentansprüche**

1.  Nadelentsorgungssystem mit einer zum Halten mit der Hand geeigneten Anordnung, wobei die Anordnung eine erste Kappe (16), eine Kopfeinheit (12) und einen Wegwerfbehälter (10) zum Aufnehmen mehrerer gebrauchter Nadeln aufweist, wobei die erste Kappe (16) eine Öffnung (30), die Kopfeinheit (12) einen durch eine Öffnungseinrichtung, welche eine Ventilvorrichtung enthält, gebildeten Durchlaß und der Behälter (10) einen in seinem oberem Ende (6) ausgebildeten Einlaß (4) aufweist, dadurch gekennzeichnet, daß die Kopfeinheit (12) zweiteilig ausgebildet ist, wobei ein erster Teil zwei voneinander beabstandete Platten (20, 20A) mit zueinander ausgerichteten Öffnungen (22, 22A) aufweist, und ein zweiter Teil ein Auslösermechanismus (28) ist, der zwischen den beiden beabstandeten Platten (20, 20A) bewegbar ist und einen zu den ausgerichteten Öffnungen (22, 22A) ausrichtbaren Durchlaß (28A) aufweist, wobei die Öffnung (30), die ausgerichteten Öffnungen (22, 22A) und der Einlaß (4) zueinander ausgerichtet sind, wenn die Komponenten zusammengefügt sind, wobei der Durchlaß (28A) des Auslösermechanismus (28) in eine nicht ausgerichtete Position vorgespannt ist, wodurch, wenn der Durchlaß (28A) in Ausrichtung zu den Öffnungen (22, 22A) bewegt ist, eine Nadel in die Öffnung (30) einführbar ist, um in den Behälter (10) zu gelangen, wobei der Behälter (10) eine Einrichtungen (44) aufweist, die eine Rückkehr der Nadel durch den Einlaß (4) verhindert.

2. Nadelentsorgungssystem nach Anspruch 1, dadurch gekennzeichnet, daß die erste Kappe (16) eine umgekehrte Schürze (32) aufweist, die sich nach außen und oben über die Seiten der Kopfeinheit (12) erstreckt, um als Schutzabdeckung für eine die Kopfeinheit (12) der Anordnung haltende Hand während des Einführens einer Nadel zu dienen.

3. Nadelentsorgungssystem nach Anspruch 2, dadurch gekennzeichnet, daß die Schürze (32) in Draufsicht länglich ist und eine Längsseite aufweist, deren Neigung steiler ist als diejenige der gegenüberliegenden Längsseite.

4. Nadelentsorgungssystem nach einem der vorhergehenden Ansprüche, dadurch gekennzeichnet, daß der Auslösermechanismus (28) federvorgespannt ist.

5. Nadelentsorgungssystem nach Anspruch 1 oder 4, dadurch gekennzeichnet, daß der Auslösermechanismus (28) zwei Platten (24, 24A) aufweist, die jeweils in gleitend verschiebbarer Beziehung zu den Platten (20, 20A) stehen und sich der Durchlaß (28A) zwischen den beiden Platten (24, 24A) erstreckt, wobei die beiden Platten (24, 24A) Öffnungen (26, 26A) aufweisen.

6. Nadelentsorgungssystem nach Anspruch 5, dadurch gekennzeichnet, daß die Ventilvorrichtung durch die Öffnung (22) der Deckplatte (20) der voneinander beabstandeten Platten (20, 20A) gebildet ist, und die Öffnung (26) in der oberen der beiden Platten (24, 24A) ausgebildet ist.

7. Nadelentsorgungssystem nach Anspruch 6, dadurch gekennzeichnet, daß die beiden Öffnungen (22, 26) der Ventilvorrichtung mit einer Reihe von Sägezahneinschnitten (62, 62A) entlang gegenüberliegenden halbkreisförmigen Teilen derselben versehen sind.

8. Nadelentsorgungssystem nach Anspruch 7, dadurch gekennzeichnet, daß in der Deckplatte und der oberen Platte (20, 24) falze zur Aufnahme von Metallplatten (60) ausgebildet sind, in welchen die Öffnungen (22, 26) mit den Sägezahneinschnitten (62, 62A) vorgesehen sind.

9. Nadelentsorgungssystem nach einem der vorhergehenden Ansprüche, dadurch gekennzeichnet, daß das Ausmaß der Bewegung des Auslösermechanismus (28) durch Schlitze (8) bestimmt ist, durch welche sich ein stationärer Stift (58) erstreckt, der in den beabstandeten Platten (20, 20A) verankert ist.

10. Nadelentsorgungssystem nach einem der Ansprüche 2 bis 9, dadurch gekennzeichnet, daß die erste Kappe (16), die Kopfeinheit (12) und der Behälter (10) zwischen benachbarten Elementen zusammenwirkende männliche und weibliche Verbindungsteile aufweisen.

11. Nadelentsorgungssystem nach einem der vorhergehenden Ansprüche, dadurch gekennzeichnet, daß die Verhinderungseinrichtung ein rohrförmiger fortsatz (44) ist, der um den Einlaß (4) des Behälters (10) herum nach innen vorsteht.

12. Nadelentsorgungssystem nach einem der vorhergehenden Ansprüche, dadurch gekennzeichnet, daß der Behälter (10) aus einem Material besteht, das dem Durchtritt einer Nadel widersteht.

13. Nadelentsorgungssystem nach einem der vorhergehenden Ansprüche, dadurch gekennzeichnet, daß die Kopfeinheit (12) und die erste Kappe (16) wiederverwendbar sind und aus einem autoklavierbaren Material bestehen.

14. Nadelentsorgungssystem nach einem der vorhergehenden Ansprüche, dadurch gekennzeichnet, daß der Behälter (10) und die Kopfeinheit (12) aus Kunststoffmaterial bestehen.

15. Nadelentsorgungssystem nach einem der vorhergehenden Ansprüche, dadurch gekennzeichnet, daß eine zweite Kappe (36) in Form eines Verschlusses vorgesehen ist, die nach Gebrauch und vor dem Entsorgen auf dem Behälter (10) anbringbar ist.

## Revendications

1. Système de mise au rebut d'aiguilles comprenant un ensemble adapté pour être tenu en main, l'ensemble comportant un premier capuchon (16), une unité de tête (12) et un récipient jetable (10) pour contenir une série d'aiguilles usagées, le premier capuchon (16) comportant une ouverture (30), l'unité de tête (12) comportant un passage formé par un moyen d'ouverture incorporant un dispositif de vanne et le récipient (10) comportant une entrée (4) prévue à son extrémité supérieure (6), caractérisé en ce que l'unité de tête (12) est formée de deux parties, une première partie comportant deux plaques espacées (20, 20A) dans lesquelles sont prévues des ouvertures alignées (22, 22A) et une seconde partie constituée par un mécanisme à poussoir (28) mobile entre les deux plaques espacées (20, 20A) et comportant un passage (28A) alignable sur les ouvertures alignées (22, 22A), l'ouverture (30) et les ouvertures alignées (22, 22A) ainsi que l'entrée (4) étant en alignement lors de l'assemblage des éléments, le passage (28A) du mécanisme à poussoir (28) étant sollicité vers une position hors d'alignement de sorte que lors du déplacement du passage (28A) dans l'alignement des ouvertures (22, 22A) une aiguille puisse être introduite dans l'ouverture (30) pour passer dans le récipient (10), le récipient (10) comportant un moyen (44) pour empêcher le retour d'une aiguille par l'entrée (4).

2. Système de mise au rebut d'aiguilles suivant la revendication 1, caractérisé en ce que le premier capuchon (16) comporte une jupe inversée (32) s'étendant vers le haut et vers l'extérieur au-delà des côtés de l'unité de tête (12) en servant ainsi de protection pour protéger la main lorsque l'on tient l'unité de tête (12) de l'ensemble précité au cours d'une opération d'introduction d'aiguille.

3. Système de mise au rebut d'aiguilles suivant la revendication 2, caractérisé en ce que la jupe (32) est allongée en plan et comporte un côté longitudinal avec une inclinaison plus forte que le côté longitudinal opposé.

4. Système de mise au rebut d'aiguilles suivant l'une quelconque des revendications précédentes, caractérisé en ce que le mécanisme à poussoir (28) est rappelé par ressort.

5. Système de mise au rebut d'aiguilles suivant l'une ou l'autre des revendications 1 et 4, caractérisé en ce que le mécanisme à poussoir comporte deux plaques (24, 24A) en relation de coulissement respectivement avec des plaques (20, 20A) et entre lesquelles deux plaques (24, 24A) s'étend le passage (28A), les deux plaques (24, 24A) comportant des ouvertures (26, 26A).

6. Système de mise au rebut d'aiguilles suivant la revendication 5, caractérisé en ce que le dispositif de vanne est formé par l'ouverture (22) de la plaque de sommet (20) des plaques espacées (20, 20A), et l'ouverture (26) de la plaque supérieure des deux plaques (24, 24A).

7. Système de mise au rebut d'aiguilles suivant la revendication 6, caractérisé en ce que les deux ouvertures (22, 26) du dispositif de vanne sont pourvues d'une série de dentelures (62, 62A) agencées autour de parties semi-circulaires opposées de celles-ci.

8. Système de mise au rebut d'aiguilles suivant la revendication 7, caractérisé en ce que des encastrements sont formés dans les plaques de sommet et supérieure (20, 24) pour loger des plaques métalliques (60) dans lesquelles sont prévues les ouvertures (22, 26) avec les dentelures (62, 62A).

9. Système de mise au rebut d'aiguilles suivant l'une quelconque des revendications précédentes, caractérisé en ce que l'ampleur du déplacement du mécanisme à poussoir (28) est déterminée par des fentes (8) au travers desquelles s'étend une broche stationnaire (58), qui est fixée dans les plaques espacées (20, 20A).

10. Système de mise au rebut d'aiguilles suivant l'une quelconque des revendications 2 à 9, caractérisé en ce que le premier capuchon (16), l'unité de tête (12) et le récipient (10) comportent des éléments de connexion mâle et femelle coopérants entre des éléments contigus.

11. Système de mise au rebut d'aiguilles suivant l'une quelconque des revendications précédentes, caractérisé en ce que le moyen d'empêchement est un prolongement tubulaire (44) faisant saillie à l'intérieur de l'entrée (4) du récipient (10).

12. Système de mise au rebut d'aiguilles suivant l'une quelconque des revendications précédentes, caractérisé en ce que le récipient (10) est fait en une matière résistant au passage d'une aiguille.

13. Système de mise au rebut d'aiguilles suivant l'une quelconque des revendications précédentes, caractérisé en ce que l'unité de tête (12) et le premier capuchon (16) sont réutilisables et sont faits en une matière pouvant résister à la mise en autoclave.

14. Système de mise au rebut d'aiguilles suivant l'une quelconque des revendications précédentes, caractérisé en ce que le récipient (10) et l'unité de tête (12) sont faits en une matière plastique.

15. Système de mise au rebut d'aiguilles suivant l'une quelconque des revendications précédentes, caractérisé en ce qu'est prévu un second capuchon (36) sous la forme d'une fermeture, fixable sur la partie supérieure du récipient (10) après utilisation et avant la mise au rebut.

# Fig.1.

# Fig.2.

# Fig.3A.

# Fig.3B.

# Fig.8.

Fig.4.

Fig.7.

Fig.5.

Fig.6.